(19) Europäisches Patentamt
European Patent Office
Office européen des brevets

(11) Publication number: **0 334 424 B1**

(12) # EUROPEAN PATENT SPECIFICATION

(45) Date of publication of patent specification: **22.12.93**

(51) Int. Cl.5: **C08K 5/34**, C07D 211/48

(21) Application number: **89200647.9**

(22) Date of filing: **15.03.89**

(54) **Stabilizer compounds and processes for preparing them.**

(30) Priority: **23.03.88 IT 1990588**

(43) Date of publication of application:
**27.09.89 Bulletin 89/39**

(45) Publication of the grant of the patent:
**22.12.93 Bulletin 93/51**

(84) Designated Contracting States:
**AT BE CH DE ES FR GB GR LI LU NL SE**

(56) References cited:

**CHEMICAL ABSTRACTS, vol. 82, no. 20, 19th May 1975, page 42, abstract no. 126151m, Columbus, Ohio, US; & SU-A-444 781**

**CHEMICAL ABSTRACTS, vol. 97, no. 1, 5th July 1982, page 591, abstract no. 6113p, Columbus, Ohio, US; L.A. MYSHKINA et al.: "New stable radical in 2,2,6,6-tetramethyl-4-piperidole", & IZV. AKAD. NAUK SSSR, SER. KHIM. 1982, (2), 328-33**

(73) Proprietor: **ENIRICERCHE S.p.A.**
**Corso Venezia 16**
**I-20121 Milan(IT)**

Proprietor: **ENICHEM SYNTHESIS S.p.A.**
**Via Ruggero Settimo 55**
**I-90139 Palermo(IT)**

(72) Inventor: **Roggero, Arnaldo**
**Via Libertà 72**
**I-20097 San Donato Milanese Milan(IT)**
Inventor: **Lezzi, Alessandro**
**Viale Monza 112**
**I-20125 Milan(IT)**
Inventor: **Bertolini, Guglielmo**
**Via Parco Vecchio 30/3**
**I-27100 Pavia(IT)**
Inventor: **Busetto, Carlo**
**Via Morandi 11/E**
**I-20097 San Donato Milanese Milan(IT)**

(74) Representative: **Roggero, Sergio et al**
**Ing. Barzanò & Zanardo Milano S.p.A.**
**Via Borgonuovo 10**
**I-20121 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to stabilizer compounds for organic polymers and to the processes for preparing them.

The invention relates also to the use of these compounds in the stabilization of organic polymers against the degradative action of light.

It is known that the organic polymers undergo degradation with time, owing to the effect of their exposure to the atmospheric agents and in particular to U.V. light.

In order to counteract such a degradation, it is customary in the art to incorporate to the organic polymers small amounts of stabilizer compounds, generally constituted by sterically hindered amines, such as disclosed, e.g., in U.S. patents Nos. 3,640,928; 3,840,494 and 4,046,731.

In the stabilization of the organic polymers with sterically hindered aminic compounds, problems exist as regards the compatibility between the stabilizer and the polymer, and the extractibility of the stabilizer from the same polymer, with this latter phenomenon also depending on the value of the molecular weight of the stabilizer used. Therefore, in the art stabilizer compounds were proposed, which have a relatively simple structure, but are capable of spontaneously transforming into compounds of complex resinous structure inside the organic polymer to which they were incorporated, as disclosed, e.g., in European patent application publ. No. 162,523.

Also stabilizer compounds of polymeric nature and having a structure similar to that of the polymer which one wants to stabilize were tested, as disclosed, e.g., in european patent applications Nos. 86201703.5 filed on October 2nd , 1986 and 87201029.3 filed on June 2nd , 1987.

In the art the need exists for having available stabilizer compounds in order to endow polymeric materials with light stability, which stabilizer compounds, besides having a high heat stability and a high compatibility with the polymer, are capable of simultaneously showing characteristics of low extractibility from the polymer, and of good mobility inside the polymeric structure. Stabilizers showing such characteristics are in fact particularly suitable for protecting manufactured articles made from polymeric materials having a high surface area, and, in general, polymeric manufactured articles requiring a high local concentration of the stabilizer agents owing to particularly exposed regions, as compared to other, less exposed, regions.

The present Applicant found now that such requirements can be fulfilled by incorporating to the organic polymers new stabilizers having a special structure.

Therefore, a purpose of the present invention is a class of stabilizers for organic polymers containing sterically hindered amino groups.

Another purpose of the present invention are the processes for preparing such stabilizers.

A further purpose of the present invention are the stabilized polymeric compositions which contain the same stabilizers.

Still further purposes of the present invention will be clear from the following disclosure.

The stabilizer compounds for organic polymers according to the present invention contain a sterically hindered amino group and can be defined by means of the general formula (I):

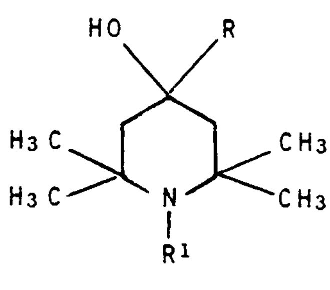

(I)

where:

- $R^1$ is the hydrogen atom, or a linear or branched $(C^1\text{-}C_5)$-alkyl group, or the benzyl group; and
- R is a primary, secondary or tertiary alkyl group containing from 16 to 20 carbon atoms.

Preferably, $R^1$ is a primary alkyl group.

Preferably, R represents either hydrogen or a methyl group.

It should be observed that the compounds of formula (I) can coexist with the relevant de-hydrated forms, having the structures:

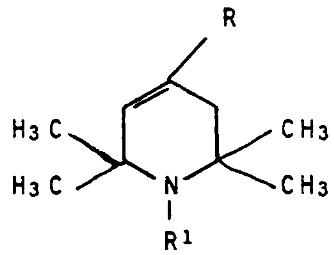

(Ia)

and

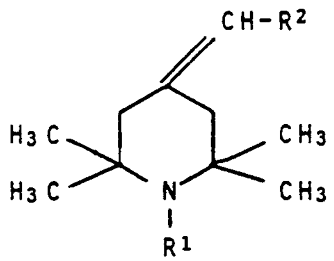

(Ib)

(where $R^2$ is an alkyl group containing one carbon atom less than the R group as hereinabove defined). Also the structures (Ia) and (Ib) are encompassed in the scope of this invention.

The compouds (I) can be obtained by means of the reaction, in a first reaction step, of an alkyl halide RX [wherein R has the same meaning as stated for formula (I) and X is a halogen atom, preferably chlorine or bromine] with two equivalents of an alkali metal M (preferably sodium or potassium), to yield the corresponding metallorganic derivative and the subsequent reaction, in a second reaction step, of such derivative with 2,2,6,6-tetramethylpiperidone, or a nitrogen-substituted derivative of this latter, according to the following reaction scheme:

$$R\text{-}X + 2\,M \rightarrow R\text{-}M + M\text{-}X$$

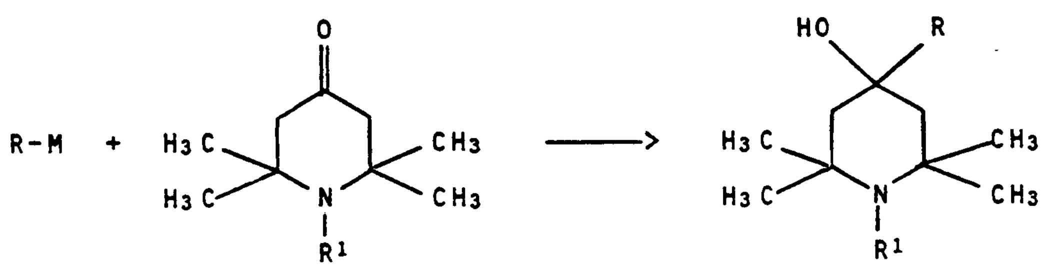

According to another route, the compounds (I) can be obtained by means of the reaction of an alkyl halide RX (wherein R and X have the above stated meaning) with an equimolecular, or nearly equimolecular, amount of an alkaline-earth metal $M^1$ (preferably magnesium) to yield the corresponding metallorganic derivative, and the subsequent reaction of this latter with an equimolecular, or nearly equimolecular, amount of 2,2,6,6-tetramethylpiperidone, or of a nitrogen-substituted derivative of this latter, according to the reaction scheme:

$$RX + M^1 \rightarrow RM^1X$$

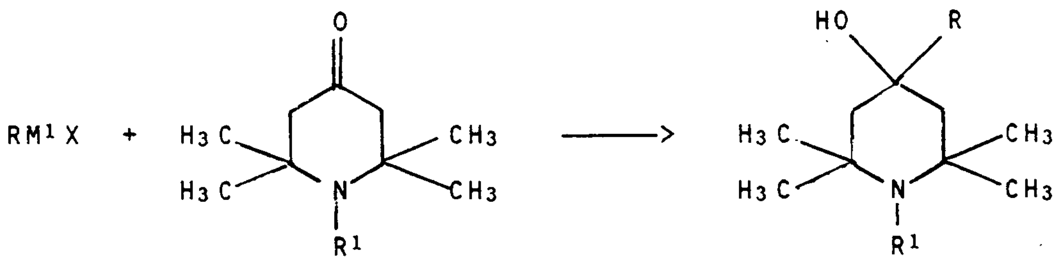

Therefore, in the process according to the present invention, the reaction is exploited of a carbanionic structure RM or $RM^1X$ (M. Szwarc, Carbanions, Living Polymers and Electron-Transfer Processes, Interscience Publishers, 1968), which structure is generated in its turn by the reaction of an alkali or alkaline-earth metal with an alkyl halide, with the ketonic function of 2,2,6,6-tetramethyl-piperidone or of an N-substituted derivative of this latter.

The structure of RM or of $RM^1X$ and hence of the alkyl halide used as the starting compound is so selected, according to the present invention, as to give the eventually obtained (I) compound that structure which is hereinabove defined, and which makes it possible the following effects to be achieved:

- the molecular weight of the compound is increased to an extent which is sufficient to limit the volatility and the extractibility of the said compound from the organic polymer;
- the compound is rendered compatible with the organic polymer it is incorporated to, in particular with an olefinic polymer;
- the compound is mobile inside the same polymer.

In accordance therewith, specific examples of suitable alkyl halides for the preparation of stabilizer compounds according to the present invention are: dodecyl chloride, dodecyl bromide, hexadecyl chloride, hexadecyl bromide, octadecyl chloride, octadecyl bromide, tetradecyl chloride and tetradecyl bromide. According to a form of practical embodiment of the present invention, such alkyl halides are obtained from the oxo-alcohols by replacing the alcoholic hydroxy group with a halogen atom, by operating according to methods known from the prior art.

One should observe that according to the present invention, the nature of the R group in the above formula (I) is critical. So, e.g., the product of reaction of a lithium-alkyl and 2,2,6,6-tetramethyl-piperidone [as reported by L.A. Myshkina, F.M. Stoyanovich, E.P. Lubruzh, M.A. Ondar and O. Ya. Gruiberg in: Izv. Akad. Nauk. SSSR. Ser. Khim. 1982, (Z), 328-333] is unsuitable for the stabilization of the organic polymers in that its volatility makes it possible only very limited times of permanence thereof in the organic polymers to be accomplished.

In the practicing of the process according to the present invention, the alkyl halide RX is suitably reacted with an alkali metal or an alkaline-earth metal suspended in an inert organic solvent such as n-heptane, cyclohexane, methyl-cyclohexane and benzene, by operating at a temperature comprised within the range of from -10 °C to 50 °C.

The so obtained reaction mixture containing the RM or $RM^1X$ compound is subsequently brought to a temperature which is preferably comprised within the range of from 10 °C to 30 °C and to it 2,2,6,6-tetramethyl-piperidone, or an N-substituted derivative thereof, is subsequently added in an amount which is stoichiometrically equivalent, or nearly stoichiometrically equivalent, to the RM or $RM^1X$ compound.

The reaction is allowed to proceed at the above indicated temperature for a time of the order of 1 hour. The reaction is then quenched by means of the addition of a small amount of methanol to the reaction mixture, and the compound (I) is separated by means of the usual separation techniques.

The stabilizer compounds (I) according to the present invention can be easily homogenized with the organic polymers and are endowed with characteristics of compatibility, mobility and non-extractibility in particular as regards the polyolefins.

Therefore, according to a further aspect thereof, the present invention relates to compositions stable to the degradative action by U.V. light, which comprise an organic polymer, in particular a polyolefin, i.e., polyethylene and polypropylene, and an amount of a stabilizer compound (I) which ensures the presence inside the composition of an amount of active nitrogen comprised within the range of from 0,005 to 0.02% by weight, and preferably of the order of 0.015% by weight.

By "active nitrogen", the nitrogen supplied by the sterically hindered amino group is herein meant.

The stabilized polymeric compositions according to the present invention can be prepared by means of any known techniques used for the purpose of homogenizing an organic polymer with the stabilizer agent.

The following experimental examples illustrate the present invention in greater detail.

Example 1

A temperature-controlled reactor of 0.5 litres of capacity is used, which is equipped with a stirrer, a charging funnel, a pressure and temperature indicator, and with a nitrogen inlet system.

Under an inert atmosphere, 200 ml of n-heptane and 45 mmol (1.04 g) of finely subdivided sodium metal are charged. By operating at a temperature of about -5 °C, 20 mmol (5.22 g) of cetyl chloride (1-chloro-n-hexadecane) is gradually added during a time of 2 hours. At the end of the addition, the temperature of the reaction mixture is allowed to rise to room temperature values (20-25 °C) and 20 mmol (3.1 g) of 2,2,6,6-tetramethyl-piperidone is added. The reaction mass is kept stirred at room temperature for 1 hour.

The reaction is stopped by pouring the reaction mixture into an aqueous solution of ammonium chloride. The aqueous phase is repeatedly extracted with ethyl ether. The so obtained organic phases are combined and washed many times with water, and the combined organic phase is are dried over anhydrous sodium sulfate and concentrated in a rotary evaporator.

The obtained residue is dissolved into ethane and through the obtained solution hydrogen chloride gas is bubbled. The precipitate formed, recovered by filtration, is poured into an aqueous solution of potassium hydroxide and the aqueous phase is extracted with ethyl ether. Ethyl ether is evaporated off and the reaction product is recovered. On gas-chromatographic analysis, the reaction product results to be pure; yield 55%.

The so obtained solid is submitted to MS and NMR analyses, and to the analytical test for nitrogen content.

It structure results to be:

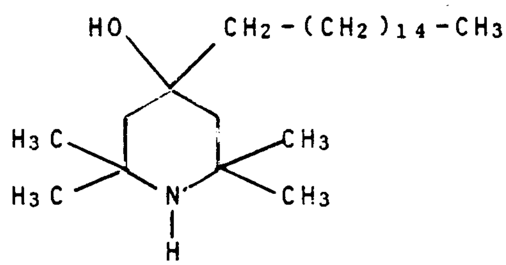

(1)

Example 2

The test of Example 1 is repeated with the difference that 1-bromo-n-octadecane is used instead of cetyl chloride.

To 200 ml of methylcyclohexane 50 mmol of finely dispersed sodium metal (1.15 g) is added, and, by operating at a temperature of -10 °C, 20 mmol of 1-bromo-n-octadecane is slowly added. At the end of the addition, the temperature is allowed to rise up to 20 °C, and 20 mmol of 1,2,2,6,6,-pentamethyl-piperidone is added. One hour later, the reaction is quenched and the product is isolated by operating in the same way as in Example 1; yield 50%.

The isolated product shows the structure:

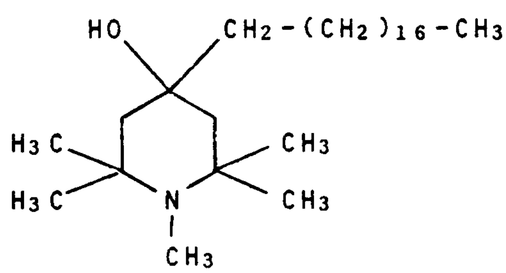

(2)

Examples 3-6

To the same equipment as of Example 1, after thoroughly drying, 44.25 milligrams-atom of magnesium and about 25 ml of benzene are charged uner a nitrogen stream. Then a solution of 35.4 mmol of alkyl halide and 70.8 mmol of ethyl ether in 25 ml of benzene is dropwise added with stirring.

The dripping is so regulated, as to maintain the reaction mixture at about 40 ° C.

When the addition is ended, the temperature is increased up to 50 ° C and the reaction mass is kept stirred for a further hour.

The so-obtained solution is cooled down to 20 ° C, and 29.7 mmol of piperidone and 40 ml of benzene are rapidly added; heat development is observed. When the addition is ended, the reaction is stopped and the product is isolated by operating according to as disclosed in Example 1.

In particular, in (comparative) Example 3, 1-chloro-n-dodecyl and 1,2,2,6,6-pentamethyl-piperidone were used as the reactants, and a compound containing 4.13% by weight of nitrogen was obtained with a yield of 70% as referred to this latter reactant. The structure of said compound resulted to be the following:

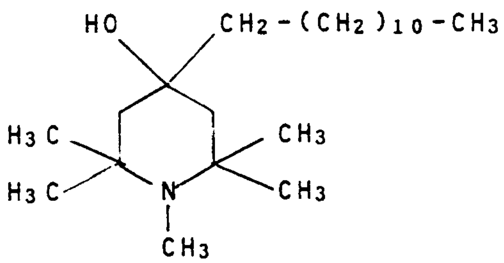

(3)

In the (comparative) Example 4, 1-chloro-n-dodecyl and 2,2,6,6-tetramethyl-piperidone were used as the reactants, and a compound containing 4.31% by weight of nitrogen was obtained with a yield of 72% as referred to this latter reactant. The structure of said compound resulted to be the following:

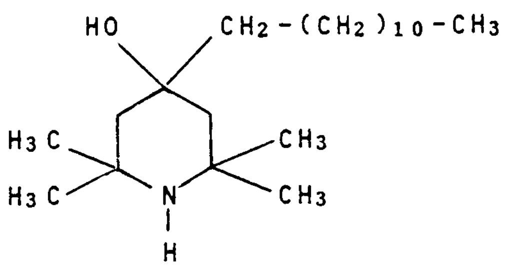

(4)

In Example 5, cetyl bromide (1-bromo-n-hexadecane) and 1,2,2,6,6-pentamethyl-piperidone were used as the reactants, and a compound containing 3.54% by weight of nitrogen was obtained with a yield of 68% as referred to this latter reactant. The structure of said compound resulted to be the following:

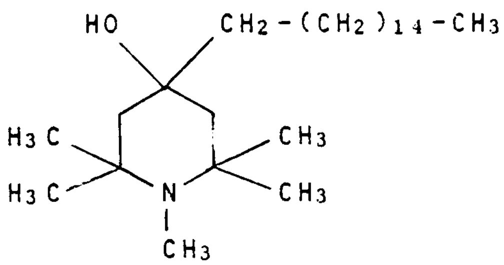

(5)

In Example 6, cetyl bromide (1-bromo-n-hexadecane) and 2,2,6,6-tetramethyl-piperidone were used as the reactants, and a compound containing 3.67% by weight of nitrogen was obtained with a yield of 68% as referred to this latter reactant. The structure of said compound resulted to be the following:

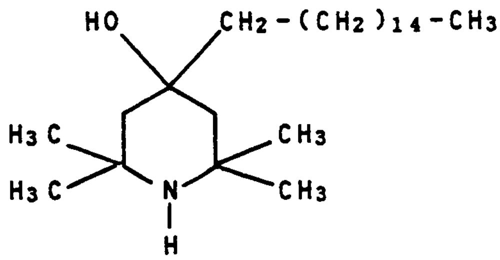

(6)

Example 7

(A) - The stabilizer agent (1) obtained in Example 1 is submitted to thermogravimetric analysis, under an inert gas atmosphere and with under programmed temperature conditions (temperature increase rate of 10 ° C/minute).

In following Table 1, the percent values of weight loss of the stabilizer agent (1), as observed within different temperature ranges, are reported as compared to the analogous values obtained with the stabilizer agent TINUVIN 770, a commercial product by CIBA-GEIGY bis-(2,2,6,6-tetramethyl-4-piperidinyl) sebacate.

TABLE 1

| Stabilizer | Weight loss | | |
|---|---|---|---|
| | T = 35-230ºC | T = 230-280ºC | T = 395ºC |
| Stabilizer agent (1) | 2.1 | 12.80 | 100.0 |
| TINUVIN 770 | 1.3 | 20.1 | 100.0 |

The stabilizer agents (4) and (3) of Examples 4 and 3 are similarly submitted to thermogravimetric analysis and the results of such determinations are respectively reported in Tables 2 and 3.

TABLE 2

(Sample 4: Comparative Sample)

| Temperature range, ºC | Weight loss (%) |
|---|---|
| 35 - 100 | 1.35 |
| 35 - 200 | 6.58 |

(cont.d) TABLE 2

(Sample 4: Comparative Sample)

| Temperature range, °C | Weight loss (%) |
|---|---|
| 35 - 250 | 11.1 |
| 35 - 300 | 31.7 |
| 35 - 350 | 98.7 |
| 35 - 400 | 100 |

TABLE 3

(Sample 3: Comparative Sample)

| Temperature range, °C | Weight loss (%) |
|---|---|
| 35 - 100 | 0.32 |
| 35 - 150 | 1.17 |
| 35 - 200 | 4.25 |
| 35 - 250 | 19.3 |
| 35 - 300 | 66.2 |
| 35 - 350 | 100 |

(B) - The stabilizing activity of the stabilizer compound (1) is verified on the basis of its ability to inhibit the degradation of polypropylene submitted to the influence of U.V. light.

The incorporation of the stabilizer compound to polypropylene is accomplished by causing this latter to swell in benzene containing the same stabilizer, and then evaporating benzene to dryness under vacuum.

In particular, to the commercial polypropylene grade FLF20 by MONTEDISON, the stabilizer compound (1) is added in such an amount as to have, in the resulting composition, an active nitrogen content of 0.015% by weight.

The resulting composition is moulded at 180 °C under a pressure of 100 kg/cm$^2$, for a time of 1.5 minutes, with a film of 100$\mu$m of thickness being obtained.

After being removed from the press, the film is rapidly quenched under tap water and is submitted to an accelerated ageing test in an UVCON equipment by ATLAS, with alternating cycles of irradiation with fluorescence lamps rich in U.V. wavelengths between 280 and 350 nm, and of condensation in the dark, under controlled-temperature conditions.

More particularly, in the herein examined case, an irradiation cycle of 8 hours at 60 °C and 4 hours of condensation at 40 °C is used.

The degradation of polypropylene is verified on the basis of the formation of carbonyl compounds (CO index), evidenced by means of the I.R. spectrum. More particularly, the CO index is determined as a function of the increase in absorption at 1710 cm$^{-1}$. In the present assay, the increase in absorption at 1710 cm$^{-1}$ is measured within a time interval of about 700 hours, on:

(1) a polypropylene film used as the reference sample [indicated by (+) in Figure 1];

(2) a polypropylene film obtained as in the preceding case, and containing the commercial stabilizer agent TINUVIN 770 in such an amount as to yield an active nitrogen content of 0.015% by weight [indicated by (△) in Figure 1];

(3) a polypropylene film obtained as in the preceding case, and containing the stabilizer agent (1) in such an amount as to yield an active nitrogen content of 0.015% by weight [indicated by (□) in Figure 1];

(4) a polypropylene film obtained as in the preceding case, and containing the stabilizer agent (3) in such an amount as to yield an active nitrogen content of 0.015% by weight [indicated by (*) in Figure

1];

(C) - The compatibility of the stabilzer compound (1) with polypropylene and its permanence in the same polymer under high thermal stress conditions are verified by measuring the steady-state concentration of the nitroxyl radical obtained from the oxidation of the sterically hindered amino group of the stabilizer molecule. Said steady-state concentration is obtained from the ESR spectra directly carried out on polymer samples containing the stabilizer agent and maintained for some hours at 170 ° C. In particular, the formation of the nitroxyl radical is directly induced in the polypropylene film by means of photo-sensitized oxidation with singlet oxygen.

The polypropylene film is obtained as hereinabove disclosed.

The amount of stabilizer agent (1) is of 2.5% by weight, corresponding to 0.015% by weight of active nitrogen.

The photosensitizer (Bengale pink, chlorophyll) is added in an amount of 0.1% by weight, as referred to the weight of the polymer. The film is then submitted to irradiation with U.V. light for 18 hours, with a high-pressure mercury vapours light ot 150 W coupled with an UV 31 filter, which ensures a radiation pass-band at 290 nm. After irradiation on a weighed portion of film, the verification is carried out - by means of ESR spectra - of the formation of the radical and of its persistence in the sample at the temperature of 170 ° C.

The film to which the stabilizer compound (1) was added resulted to be stable for 5 hours at 170 ° C, as the film did, to which TINUVIN 770 was added.

(D) - Samples of polypropylene film are prepared according to as disclosed in the above (B) chapter, which have a thickness of 110 μm, and contain the stabilizer compounds (3), (4), (5) and (6) in such amounts as to supply a content of active nitrogen of 0.015% by weight. Also a film containing the same percentage of active nitrogen is prepared by means of the addition of TINUVIN 770 [sample (7)].

Each sample is subdivided into two portions, and one of said portions [respectively denominated (3a), (4a), (5a), (6a) and (7a) samples] is submitted to an extraction test, carried out for 5 hours at 60 ° C, with an aqueous solution containing 0.5% by weight of an alkaline detergent (commercial product DIXAN by HENKEL).

Both treated and untreated samples are submitted to an ageing test in UVCON by means of the same modalities as disclosed at chapter (B).

In the chart of Figure 2, the exposure time, expressed as hours, and required in order to have a carbonyl index of 0.2 in the polypropylenic film is reported. In this chart, the samples (3), (3a), (4), (4a), (7) and (7a) are comparative samples.

**Claims**

**Claims for the following Contracting States : AT, BE, CH, LI, DE, FR, GR, GB, LU, NL, SE**

1. Sterically hindered amino-group-containing stabilizing compounds for organic polymers having the general formula:

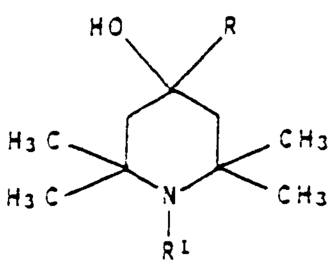

(I)

wherein:
$R^1$ is selected from a hydrogen atom, a linear or a branched $C_1$ -$C_5$ alkyl or benzyl, and
R is selected from the $C_{16}$-$C_{20}$ primary, secondary, or tertiary alkyls.

2. Compounds according to Claim 1, wherein $R^1$ is a primary alkyl.

3. Compounds according to Claim 1, wherein $R^1$ is selected from hydrogen and methyl.

4. Process for preparing the compounds (I) according to Claim 1, comprising the steps of:
(a) Preparing a metallic-organic compound having the formula:

RM or $RM^1X$

wherein M is an alkali metal and $M^1$ is an alkaline earth metal, by reacting in an inert organic solvent, at a temperature of from -10 °C to +50 °C, an alkyl halide RX, wherein X is a halogen selected from chlorine and bromine, with two equivalents of an alkali metal M or an equimolecular amount of an alkaline earth metal $M^1$, respectively; and
(b) reacting, at a temperature of from 10 °C to 30 °C, in an inert organic solvent, equimolecular amounts of either RM, or $RM^1X$, respectively, and of unsubstituted or nitrogen-substituted 2,2,6,6-tetramethylpiperidone.

5. Process according to Claim 4, wherein M is selected from sodium or potassium.

6. Process according to Claim 4, wherein $M^1$ is magnesium.

7. Stabilized polymer compositions containing an organic polymer and a stabilizing amount of a stabilizer compound (I) defined in Claim 1, wherein the stabilizing amount of said stabilizing compound corresponds to an amount of nitrogen supplied by the sterically hindered amino group in the range of from 0,005 to 0,02% by weight relative to the polymer to be stabilized.

8. Stabilized polymer compositions according to Claim 7, wherein the organic polymer is a polyolefin.

9. Stabilized polymer compositions according to Claim 8, wherein the organic polymer is polypropylene.

**Claims for the following Contracting State : ES**

1. Process for preparing sterically hindered amino-group-containing stabilizing compounds for organic polymers, having the general formula:

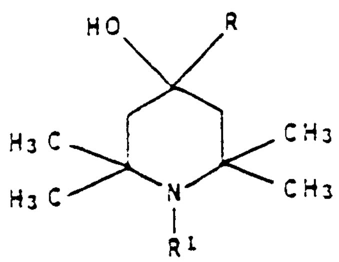

(I)

wherein:
$R^1$ is selected from a hydrogen atom, a linear or a branched $C_1$-$C_5$ alkyl or benzyl , and
R is selected from the $C_{16}$-$C_{20}$ primary, secondary, or tertiary alkyls, characterized in that it comprises the steps of:
(a) Preparing a metallic-organic compound having the formula:

RM or $RM^1X$

wherein M is an alkali metal and $M^1$ is an alkaline earth metal, by reacting in an inert organic solvent, at a temperature of from -10 °C to +50 °C, an alkyl halide RX, wherein X is a halogen selected from chlorine and bromine, with two equivalents of an alkali metal M or an equimolecular amount of an alkaline earth metal $M^1$, respectively; and
(b) reacting, at a temperature of from 10 °C to 30 °C, in an inert organic solvent, equimolecular amounts of either RM, or $RM^1X$, respectively, and of unsubstituted-, or nitrogen-substituted 2,2,6,6-tetramethylpiperidone.

2. Process according to Claim 1, wherein $R^1$ is a primary alkyl.

**3.** Process according to Claim 1, characterized in that $R^1$ is selected from hydrogen and methyl.

**4.** Process according to Claim 1, characterized in that M is selected from sodium or potassium.

**5.** Process according to Claim 1, characterized in that $M^1$ is magnesium.

**Patentansprüche**
**Patentansprüche für folgende Vertragsstaaten : AT, BE, CH, LI, DE, FR, GB, GR, LU, NL, SE**

**1.** Stabilisatorverbindungen mit einem Gehalt an einer sterisch gehinderten Aminogruppe für organische Polymere mit der allgemeinen Formel:

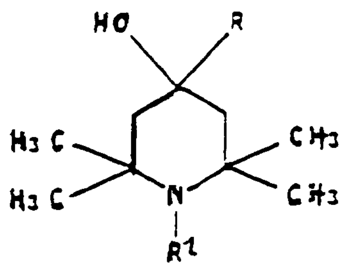

(I),

worin

$R^1$ unter einem Wasserstoffatom, geradem oder verzweigtem $C_1$-$C_5$Alkyl oder Benzyl ausgewählt ist und

R unter primärem, sekundärem oder tertiärem $c_{16}$-$C_{20}$Alkyl ausgewählt ist.

**2.** Verbindungen nach Anspruch 1, worin $R^1$ ein primäres Alkyl darstellt.

**3.** Verbindungen nach Anspruch 1, worin $R^1$ unter Wasserstoff und Methyl ausgewählt ist.

**4.** Verfahren zur Herstellung der Verbindungen (I) nach Anspruch 1, umfassend die folgenden Stufen:

(a) Herstellen einer metallorganischen Verbindung mit der Formel:

RM oder $RM^1X$,

worin M ein Alkalimetall bezeichnet und $M^1$ ein Erdalkalimetall darstellt, durch Umsetzen eines Alkylhalogenids RX, worin X ein unter Chlor und Brom ausgewähltes Halogen ist, mit zwei Äquivalenten eines Alkalimetalles M bzw. mit einer äquimolaren Menge eines Erdalkalimetalles $M^1$ in einem inerten organischen Lösungsmittel bei einer Temperatur von -10 °C bis +50 °C; und

(b) Umsetzen von äquimolaren Mengen an RM bzw. $RM^1X$ mit unsubstituiertem oder Stickstoff-substituzertem 2,2,6,6-Tetramethylpiperidon.

**5.** Verfahren nach Anspruch 4, worin M unter Natrium und Kalium ausgewählt wird.

**6.** Verfahren nach Anspruch 4, worin $M^1$ Magnesium bedeutet.

**7.** Stabilisierte Polymerzusammensetzungen mit einem Gehalt an einem organischen Polymer und einer stabilisierenden Menge einer Stabilisatorverbindung (I), wie in Anspruch 1 definiert, worin die stabilisierende Menge der Stabilisatorverbindung einer von der sterisch gehinderten Aminogruppe gelieferten Stickstoffmenge im Bereich von 0,005 bis 0,02 Gew.-%, bezogen auf das zu stabilisierende Polymer, entspricht.

**8.** Stabilisierte Polymerzusammensetzungen nach Anspruch 7, worin das organische Polymer ein Polyolefin ist.

**9.** Stabilisierte Polymerzusammensetzungen nach Anspruch 8, worin das organische Polymer Polypropylen ist.

**Patentansprüche für folgenden Vertragsstaat : ES**

**1.** Verfahren zur Herstellung von Stabilisatorverbindungen mit einem Gehalt an einer sterisch gehinderten Aminogruppe für organische Polymere, mit der allgemeinen Formel:

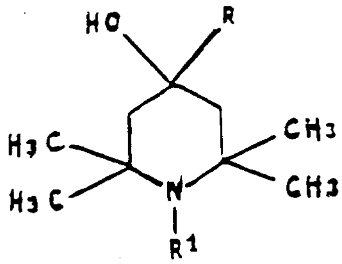

(I)

worin:

$R^1$ unter einem Wasserstoffatom, geradem oder verzweigtem $C_1$-$C_5$ Alkyl oder Benzyl ausgewählt ist und

R unter primärem, sekundärem oder tertiärem $C_{16}$-$C_{20}$ Alkyl ausgewählt ist, dadurch gekennzeichnet, daß es die folgenden Stufen umfaßt:

(a) Herstellen einer metallorganischen Verbindung mit der Formel:

RM oder $RM^1X$,

worin M ein Alkalimetall bezeichnet und $M^1$ ein Erdalkalimetall darstellt, durch Umsetzen eines Alkylhalogenids RX, worin X ein unter Chlor und Brom ausgewähltes Halogen ist, mit zwei Äquivalenten eines Alkalimetalles M bzw. mit einer äquimolaren Menge eines Erdalkalimetalles $M^1$ in einem inerten organischen Lösungsmittel bei einer Temperatur von -10 °C bis +50 °C; und

(b) Umsetzen von äquimolaren Mengen an RM bzw. $RM^1X$ mit unsubstituiertem oder Stickstoff-substituiertem 2,2,6,6-Tetramethylpiperidon.

**2.** Verfahren nach Anspruch 1, worin $R^1$ ein primäres Alkyl ist.

**3.** Verfahren nach Anspruch 1, dadurch gekennzeichnet, daß $R^1$ unter Wasserstoff und Methyl ausgewählt wird.

**4.** Verfahren nach Anspruch 1, dadurch gekennzeichnet, daß M unter Natrium und Kalium ausgewählt wird.

**5.** Verfahren nach Anspruch 1, dadurch gekennzeichnet, daß $M^1$ Magnesium ist.

**Revendications**

**Revendications pour les Etats contractants suivants : AT, BE, CH, LI, DE, FR, GR, GB, LU, NL, SE**

**1.** Composés stabilisants pour polymères organiques, contenant un groupe amino stériquement encombré et possédant la formule générale :

(I)

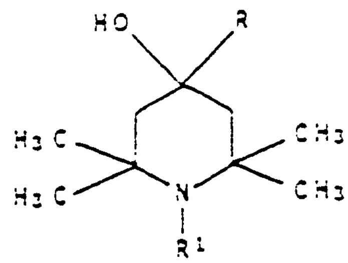

dans laquelle
$R^1$ est choisi parmi un atome d'hydrogène, un groupe alkyle en $C_1$-$C_5$ linéaire ou ramifié et un groupe benzyle, et
R est choisi parmi les groupes alkyles primaires, secondaires ou tertiaires en $C_{16}$-$C_{20}$.

**2.** Composés conformes à la revendication 1, dans lesquels $R^1$ est un groupe alkyle primaire.

**3.** Composés conformes à la revendication 1, dans lesquels $R^1$ est choisi parmi un atome d'hydrogène et un groupe méthyle.

**4.** Procédé de préparation de composés (I) conformes à la revendication 1, comportant les étapes consistant à :

a) préparer un composé organo-métallique de formule

RM ou $RM^1X$

où M représente un métal alcalin et $M^1$ représente un métal alcalino-terreux,
en faisant réagir dans un solvant organique inerte, à une température de -10°C à +50°C, un halogénure d'alkyle RX, où X représente un atome d'un halogène choisi parmi le chlore et le brome, avec deux équivalents d'un métal alcalin M, ou avec une quantité équimolaire d'un métal alcalino-terreux $M^1$, respectivement ; et

b) faire réagir, à une température de 10°C à 30°C, dans un solvant organique inerte, des quantités équimolaires de RM ou $RM^1X$, respectivement, et d'une 2,2,6,6-tétraméthylpipéridone non substituée ou substituée sur l'atome d'azote.

**5.** Procédé conforme à la revendication 4, dans lequel M est choisi parmi le sodium et le potassium.

**6.** Procédé conforme à la revendication 4, dans lequel $M^1$ est le magnésium.

**7.** Compositions stabilisées de polymères, comprenant en combinaison un polymère organique et une quantité stabilisatrice d'un composé stabilisant (I) défini dans la revendication 1, dans lesquelles la quantité stabilisatrice dudit composé stabilisant correspond à une quantité d'azote, apportée par le groupe amino stériquement encombré, située dans l'intervalle allant de 0,005 à 0,02 % en poids par rapport au polymère à stabiliser.

**8.** Compositions stabilisées de polymères, conformes à la revendication 7, dans lesquelles le polymère organique est une polyoléfine.

**9.** Compositions stabilisées de polymères, conformes à la revendication 8, dans lesquelles le polymère organique est un polypropylène.

**Revendications pour l'Etat contractant suivant : ES**

**1.** Procédé de préparation de composés stabilisants pour polymères organiques, contenant un groupe amino stériquement encombré et possédant la formule générale :

(I)

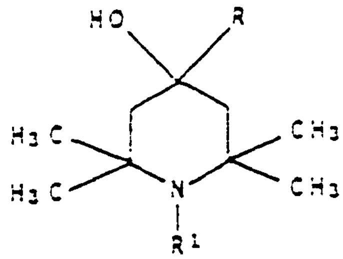

dans laquelle
$R^1$ est choisi parmi un atome d'hydrogène, un groupe alkyle en $C_1$-$C_5$ linéaire ou ramifié et un groupe benzyle, et
R est choisi parmi les groupes alkyles primaires, secondaires ou tertiaires en $C_{16}$-$C_{20}$,
caractérisé en ce qu'il comporte les étapes consistant à :

a) préparer un composé organo-métallique de formule

RM ou $RM^1X$

où M représente un métal alcalin et $M^1$ représente un métal alcalino-terreux,
en faisant réagir dans un solvant organique inerte, à une température de -10 °C à +50 °C, un halogénure d'alkyle RX, où X représente un atome d'un halogène choisi parmi le chlore et le brome, avec deux équivalents d'un métal alcalin M, ou avec une quantité équimolaire d'un métal alcalino-terreux $M^1$, respectivement ; et

b) faire réagir, à une température de 10 °C à 30 °C, dans un solvant organique inerte, des quantités équimolaires de RM ou $RM^1X$, respectivement, et d'une 2,2,6,6-tétraméthylpipéridone non substituée ou substituée sur l'atome d'azote.

**2.** Procédé conforme à la revendication 1, dans lequel $R^1$ est un groupe alkyle primaire.

**3.** Procédé conforme à la revendication 1, caractérisé en ce que $R^1$ est choisi parmi un atome d'hydrogène et un groupe méthyle.

**4.** Procédé conforme à la revendication 1, caractérisé en ce que M est choisi parmi le sodium et le potassium.

**5.** Procédé conforme à la revendication 1, caractérisé en ce que $M^1$ est le magnésium.

Fig.1

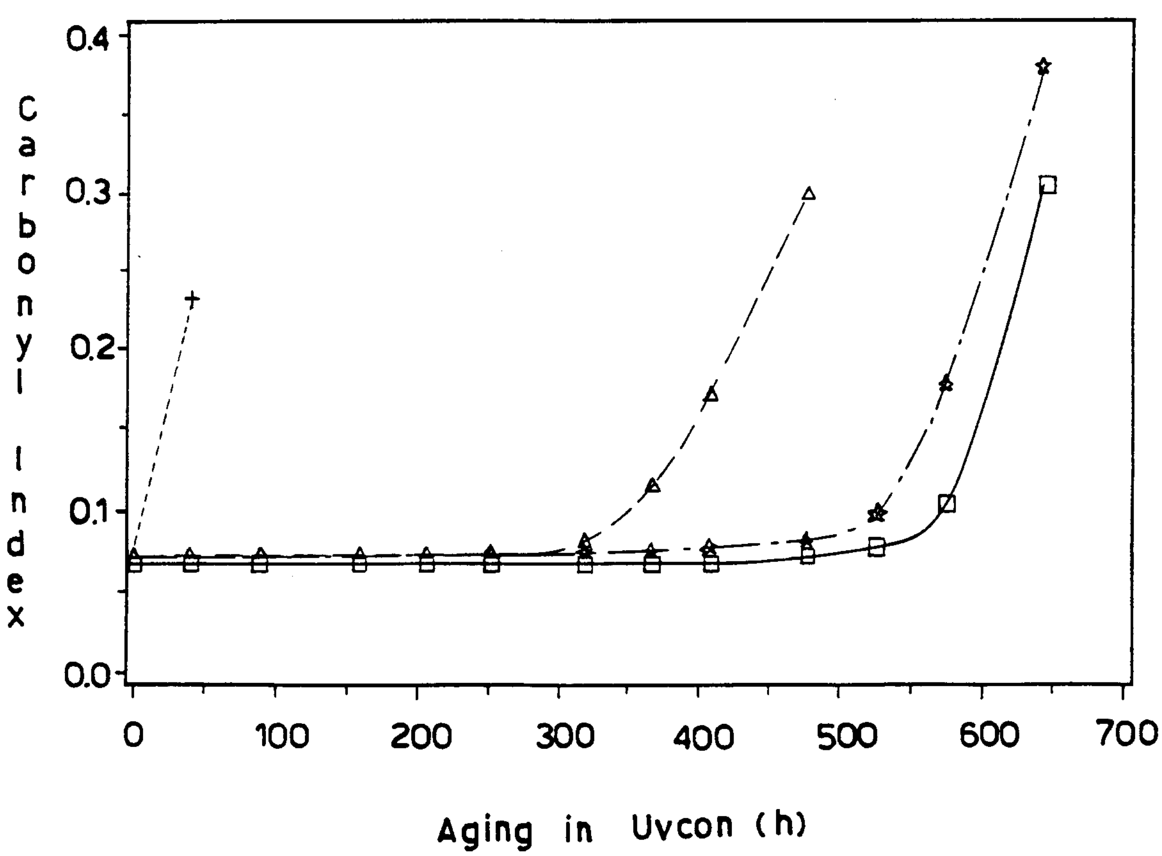
0.4
0.3
0.2
0.1
0.0
Carbonyl Index
0
100
200
300
400
500
600
700
Aging in Uvcon (h)

Fig. 2

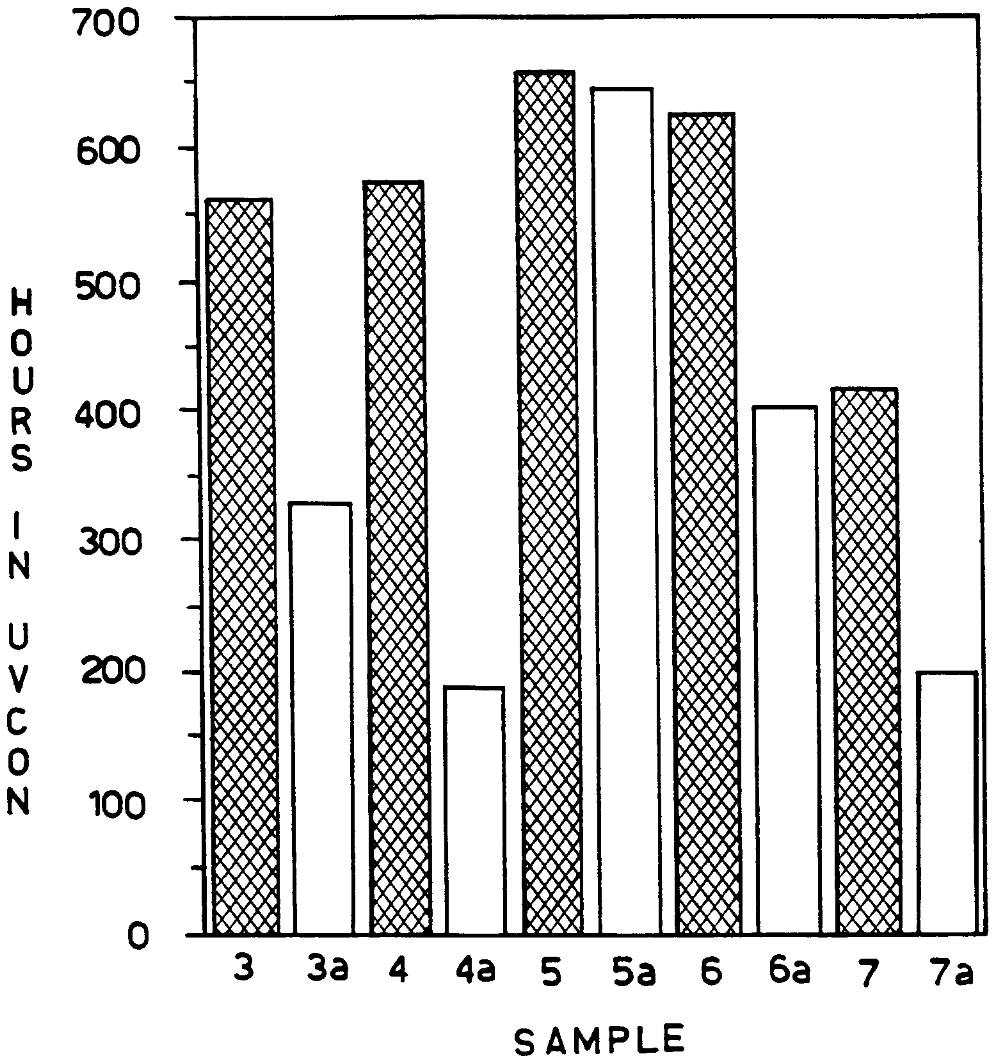
700
600
500
400
300
200
100
0
HOURS IN UVCON
3
3a
4
4a
5
5a
6
6a
7
7a
SAMPLE